# EUROPEAN PATENT APPLICATION

(11) **EP 4 625 418 A1**
(43) Date of publication of application: **01.10.2025**
(21) Application number: 25165671.6
(22) Date of filing: 24.03.2025
(51) Int. Cl.: G16B 15/30, G16B 40/20, G16C 20/50

(54) **IDENTIFYING CANDIDATE BIORECEPTORS USING COMBINED MOLECULAR DYNAMICS (MD) SIMULATIONS AND ARTIFICIAL NEURAL NETWORK BASED SCREENING**

(30) Priority: 27.03.2024 IN 202421024491
(71) Applicant: Tata Consultancy Services Limited, Maharashtra (IN)
(72) Inventor: DESHPANDE, Parijat Dilip, 411028 Pune (IN); RAI, Beena, 411028 Pune, Maharashtra (IN); BADHE, Yogesh Kailas, 411028 Pune (IN); KUMAR, Dharmendr, 411057 Pune (IN)
(74) Representative: Goddar, Heinz J.

(57) **Abstract**

This disclosure relates generally to a method and system for identifying candidate bioreceptors suitable for designing biosensors to identify an analyte of interest. State-of-art methods mainly focus on interaction of the bioreactor and the analyte. However, interaction of the bioreceptor with a substrate before binding to the analyte as well as a biofluid surrounding the bioreceptor and the analyte greatly influences such interactions. The present method utilizes combined approach of molecular dynamics (MD) and artificial neural network (ANN) based screening to systematically identify candidate bioreceptor with favorable energy profile and feasible interactions with the analyte of interest. The method involves computing RMSD plots to study individual interactions among a bioreceptor-substrate, a bioreceptor-analyte and an analyte-bioreceptor-substate complex. Further, potential of mean force (PMF) is computed for the analyte-bioreceptor-substate complex. The RMSD plots and PMF features are fed to an ANN model that predicts the suitable candidate bioreceptors through feature engineering.

## Description

### CROSS-REFERENCE TO RELATED APPLICATIONS AND PRIORITY

The present application claims priority from Indian patent application no. 202421024491, filed on March 27, 2024.

### TECHNICAL FIELD

The disclosure herein generally relates to biosensors, and, more particularly, to systems and methods for designing biosensors by identifying candidate bioreceptors for an analyte of interest.

### BACKGROUND

The identification of sensitive, accurate biosensors that are capable of real-time analysis of various biomarkers in a biofluid is one important potential feature of the recent technological development in the healthcare sector. Detection and quantification of biomarkers like glucose, cortisol, testosterone etc. in a biofluid such as eccrine sweat offers a noninvasive and potentially real-time method for monitoring physiological state of a person. Development of biosensors is traditionally conducted by various experimental methods and is therefore significantly more time consuming, expensive and has limitations in sample space and number of experiments. However, computational design of biosensors has presented a way to systematically identify a suitable bioreceptor for a particular analyte of interest by using reliable structure prediction, stability of the structure, and accurate descriptors of molecular interactions. The computational methods to guide the biosensor design, includes molecular dynamics (MD) simulations, quantum mechanics (QM) calculations, molecular docking, and a combination of them as the hybrid methodologies. The MD simulation is a potent computational method to investigate the structure, dynamics, and energetics of the biological systems in the diverse experimental (pressure, temperature, pH, and varying salt concentrations) conditions. The MD simulations can obtain key information such as thermodynamic properties at the atomistic levels and are extremely advantageous in providing physical and chemical intuition for designing biosensors with the highest efficiency. Root mean square deviation (RMSD), a numerical measurement representing the difference between atoms, backbones, chains in a bioreceptor (i.e. a protein), and free energy calculations of a potential of mean force (PMF) based on the combination of MD simulations and umbrella samplings as a function of physical coordinates have been applied to explore the detailed pathways and the corresponding free energy profiles for designing the biosensors. The MD simulations can be an advantageous method to investigate the effect of the changes of bioreceptor components on the target binding affinity. However, biggest challenge in utilizing MD techniques is to handle interaction between biomolecules and substrate. Handling such interaction in the MD system requires precise understanding of atomic level interactions and focusing on those interactions that majorly impact an association of the analyte with the bioreceptor. While studying the bioreceptor in a physiological system, challenge is to adopt a multiparametric approach that not only focuses on binding energy related calculations between analyte and the bioreceptor, but it also considers effect of surroundings (such as biofluid / solvent),interaction with the substrate supporting the bioreceptor as well as multi-stage and multi-level RMSD calculations.

### SUMMARY

Embodiments of the present disclosure present technological improvements as solutions to one or more of the above-mentioned technical problems recognized by the inventors in conventional systems. For example, in one embodiment, a method for identifying candidate bioreceptors suitable for designing biosensors to identify an analyte of interest is provided. The method includes receiving, via one or more hardware processors, an analyte, and a plurality of bioreceptors, wherein the bioreceptors are selected from a bioreceptor library based on their known affinity to the analyte. The analyte selection is modelled based on its concentration of the chemical in the biological fluid to be detected from a biosensor. The analyte is further processed for making it suitable for MD simulation. Further, the plurality of bioreceptors are obtained from a bioreceptor library based on their known affinity to the analyte. The plurality of bioreceptors with known affinity towards the analyte are extracted from public repository. The plurality of bioreceptors selected based on the binding affinity are processed by applying edits to curate the structure, orientation, and charges. The method further includes performing, via one or more hardware processors, a molecular dynamics (MD) simulation for the analyte and the plurality of bioreceptors in an explicit solvent environment to obtain: (a) a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors; (b) a bioreceptor-analyte complex by simulating each bioreceptor of the first sub-set with the analyte to obtain a second sub-set of the plurality of the bioreceptors; and (c) an analyte-bioreceptor-substrate complex by simulating the analyte, the pre-processed substrate and the second sub-set of the plurality of the bioreceptors to obtain one or more stable analyte-bioreceptor-substate complex. The surface minimization of the substrate utilized in the immobilizing the bioreceptor is performed by minimizing the layers then subjecting the minimized layers to NVT dynamics to check its stability under dynamic conditions. This energy minimized substrate interacts with one or more bioreceptors individually. This is performed to ensure that the minimized substrate is compatible with the first sub-set of the plurality of bioreceptors received. Next, the bioreceptor-analyte complex is obtained by simulating each bioreceptor of the first sub-set with the analyte. The binding interactions between each bioreceptor of the first sub-set and the analyte is assessed that involves binding affinity and specificity calculations. The bioreceptors capable of forming favorable interactions with the analyte forms the second sub-set of the plurality of bioreceptors received. Similarly interactions with other interfering species are also determined. The analyte and the bioreceptor, duly processed and the minimized substrate are then simulated to obtain an analyte-bioreceptor-substrate complex. The method further includes computing, via one or more hardware processors, root mean square deviation (RMSD) plots of: (a) the bioreceptor-substrate complex, (b) the bioreceptor-analyte complex, and (c) the analyte-bioreceptor-substrate complex. The RMSD plots serves as a key metric to quantify the deviation of a molecular structure from its initial configuration and indicate stability. The correlation between the minimal changes in bioreceptor conformation, and the consistently low RMSD values presented reinforces the stability of the bioreceptor immobilized on the gold surface in the simulated solvent environment. The method further includes computing, via one or more hardware processors, potential of mean force (PMF) plot for the analyte-bioreceptor-substrate complex. The PMF in the MD simulations assesses the energy changes as a function of specific reaction coordinate parameter like the energy changes as a function of the distance between two residues, or as a force required to pull the ligand / molecule apart from the bioreceptor. By computing local energy barriers along a binding/unbinding path, the PMF provides thermodynamic details for molecular recognition. The method further includes predicting, via one or more hardware processors, candidate bioreceptors by processing the RMSD plots and the PMF plot features fed to a pre-trained ANN model to identify one or more candidate bioreceptor to the analyte through feature engineering. The pre-trained ANN model estimates overall structural stability and binding pocket stability of the one or more desired bioreceptor to the analyte through feature engineering to predict candidate bioreceptors from the second sub-set of the plurality of bioreceptors.

In another aspect, a system for identifying candidate bioreceptors is provided. The system includes at least one memory storing programmed instructions; one or more Input /Output (I/O) interfaces; and one or more hardware processors, a molecular dynamics (MD) model comprising analyte module and bioreceptor module, and an ANN model, operatively coupled to a corresponding at least one memory, wherein the system is configured to receive, via one or more hardware processors, an analyte, and a plurality of bioreceptors, wherein the bioreceptors are selected from a bioreceptor library based on their known affinity to the analyte. The analyte selection is modelled based on its concentration of the chemical in the biological fluid to be detected from a biosensor. The analyte is further processed for making it suitable for MD simulation. Further, the plurality of bioreceptors are obtained from a bioreceptor library based on their known affinity to the analyte. The plurality of bioreceptors with known affinity towards the analyte are extracted from public repository. The plurality of bioreceptors selected based on the binding affinity are processed by applying edits to curate the structure, orientation, and charges. Further, system is configured to perform, via one or more hardware processors, a molecular dynamics (MD) simulation for the analyte and the plurality of bioreceptors in an explicit solvent environment to obtain: (a) a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors; (b) a bioreceptor-analyte complex by simulating each bioreceptor of the first sub-set with the analyte to obtain a second sub-set of the plurality of the bioreceptors; and (c) an analyte-bioreceptor-substrate complex by simulating the analyte, the pre-processed substrate and the second sub-set of the plurality of the bioreceptors to obtain one or more stable analyte-bioreceptor-substate complex. The surface minimization of the substrate utilized in the immobilizing the bioreceptor is performed by minimizing the layers then subjecting the minimized layers to NVT dynamics to check its stability under dynamic conditions. This energy minimized substrate interacts with one or more bioreceptors individually. This is performed to ensure that the minimized substrate is compatible with the first sub-set of the plurality of bioreceptors received. Next, the bioreceptor-analyte complex is obtained by simulating each bioreceptor of the first sub-set with the analyte. The binding interactions between each bioreceptor of the first sub-set and the analyte is assessed that involves binding affinity and specificity calculations. The bioreceptors capable of forming favorable interactions with the analyte forms the second sub-set of the plurality of bioreceptors received. Similarly interactions with other interfering species are also determined. The analyte and the bioreceptor, duly processed and the minimized substrate are then simulated to obtain an analyte-bioreceptor-substrate complex. Further, system is configured to compute, via one or more hardware processors, root mean square deviation (RMSD) plots of: (a) the bioreceptor-substrate complex, (b) the bioreceptor-analyte complex, and (c) the analyte-bioreceptor-substrate complex. The RMSD plots serves as a key metric to quantify the deviation of a molecular structure from its initial configuration and indicate stability. The correlation between the minimal changes in bioreceptor conformation, and the consistently low RMSD values presented reinforces the stability of the bioreceptor immobilized on the gold surface in the simulated solvent environment. Further, system is configured to compute, via one or more hardware processors, potential of mean force (PMF) plot for the analyte-bioreceptor-substrate complex. The PMF in the MD simulations assesses the energy changes as a function of specific reaction coordinate parameter like the energy changes as a function of the distance between two residues, or as a force required to pull the ligand / molecule apart from the bioreceptor. By computing local energy barriers along a binding/unbinding path, the PMF provides thermodynamic details for molecular recognition. Further, system is configured to predict, via one or more hardware processors, candidate bioreceptors by processing the RMSD plots and the PMF plot features fed to a pre-trained ANN model to identify one or more candidate bioreceptor to the analyte through feature engineering. The pre-trained ANN model estimates overall structural stability and binding pocket stability of the one or more desired bioreceptor to the analyte through feature engineering to predict candidate bioreceptors from the second sub-set of the plurality of bioreceptors.

In yet another aspect, a computer program product including a non-transitory computer-readable medium having embodied therein a computer program for identifying candidate bioreceptors is provided. The computer readable program, when executed on a computing device, causes the computing device to receive, an analyte, and a plurality of bioreceptors, wherein the bioreceptors are selected from a bioreceptor library based on their known affinity to the analyte. The analyte selection is modelled based on its concentration of the chemical in the biological fluid to be detected from a biosensor. The analyte is further processed for making it suitable for MD simulation. Further, the plurality of bioreceptors are obtained from a bioreceptor library based on their known affinity to the analyte. The plurality of bioreceptors with known affinity towards the analyte are extracted from public repository. The plurality of bioreceptors selected based on the binding affinity are processed by applying edits to curate the structure, orientation, and charges. The computer readable program, when executed on a computing device, causes the computing device to perform, a molecular dynamics (MD) simulation for the analyte and the plurality of bioreceptors in an explicit solvent environment to obtain: (a) a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors; (b) a bioreceptor-analyte complex by simulating each bioreceptor of the first sub-set with the analyte to obtain a second sub-set of the plurality of the bioreceptors; and (c) an analyte-bioreceptor-substrate complex by simulating the analyte, the pre-processed substrate and the second sub-set of the plurality of the bioreceptors to obtain one or more stable analyte-bioreceptor-substate complex. The surface minimization of the substrate utilized in the immobilizing the bioreceptor is performed by minimizing the layers then subjecting the minimized layers to NVT dynamics to check its stability under dynamic conditions. This energy minimized substrate interacts with one or more bioreceptors individually. This is performed to ensure that the minimized substrate is compatible with the first sub-set of the plurality of bioreceptors received. Next, the bioreceptor-analyte complex is obtained by simulating each bioreceptor of the first sub-set with the analyte. The binding interactions between each bioreceptor of the first sub-set and the analyte is assessed that involves binding affinity and specificity calculations. The bioreceptors capable of forming favorable interactions with the analyte forms the second sub-set of the plurality of bioreceptors received. Similarly interactions with other interfering species are also determined. The analyte and the bioreceptor, duly processed and the minimized substrate are then simulated to obtain an analyte-bioreceptor-substrate complex. The computer readable program, when executed on a computing device, causes the computing device to compute, root mean square deviation (RMSD) plots of: (a) the bioreceptor-substrate complex, (b) the bioreceptor-analyte complex, and (c) the analyte-bioreceptor-substrate complex. The RMSD plots serves as a key metric to quantify the deviation of a molecular structure from its initial configuration and indicate stability. The correlation between the minimal changes in bioreceptor conformation, and the consistently low RMSD values presented reinforces the stability of the bioreceptor immobilized on the gold surface in the simulated solvent environment. The computer readable program, when executed on a computing device, causes the computing device to compute potential of mean force (PMF) plot for the analyte-bioreceptor-substrate complex. The PMF in the MD simulations assesses the energy changes as a function of specific reaction coordinate parameter like the energy changes as a function of the distance between two residues, or as a force required to pull the ligand / molecule apart from the bioreceptor. By computing local energy barriers along a binding/unbinding path, the PMF provides thermodynamic details for molecular recognition. The computer readable program, when executed on a computing device, causes the computing device to predict candidate bioreceptors by processing the RMSD plots and the PMF plot features fed to a pre-trained ANN model to identify one or more candidate bioreceptor to the analyte through feature engineering. The pre-trained ANN model estimates overall structural stability and binding pocket stability of the one or more desired bioreceptor to the analyte through feature engineering to predict candidate bioreceptors from the second sub-set of the plurality of bioreceptors.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of a system for identifying candidate bioreceptors using artificial neural network (ANN)based screening of a sub-set of bioreceptors obtained from molecular dynamics (MD) simulation, according to some embodiments of the present disclosure.
FIG. 2 is a diagram that illustrates essential components of the system 100 with sequential functionalities for identifying candidate bioreceptors using combined ensemble of molecular dynamics and ANN, according to some embodiments of the present invention.
FIGS. 3A and 3B are flow diagrams of an illustrative method for identifying candidate bioreceptors using MD simulation and ANN based classification, using the system of FIG. 1, according to some embodiments of the present disclosure.
FIG. 4 illustrate a candidate bioreceptor-based device sensing a biomarker in a test sample, according to some embodiments of the present disclosure.
FIGS. 5A-5B shows initial and final conformations of a candidate bioreceptor (1HKC 172 : 294) on the gold substrate surface, according to some embodiments of the present disclosure.
FIG. 6 is a candidate bioreceptor (1HKC 172 : 294) complexed with glucose analyte, according to some embodiments of the present disclosure.
FIG. 7 is a Root Mean Square Deviation (RMSD) plot of the candidate bioreceptor (1HKC 172 : 294) on the gold substrate surface, according to some embodiments of the present disclosure.
FIG. 8 is a potential of mean force (PMF) plot of an analyte with the with bioreceptor obtained by the umbrella sampling technique, according to some embodiments of the present disclosure.
FIG. 9 is a RMSD plot of analyte-bioreceptor-substrate complex at 10 ns simulation, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As used herein, the terms "analyte" "biomarker" and "ligand" are used interchangeably and refer to a chemical compound to be detected in the test sample using the biosensor designed as per the method disclosed in the present disclosure. For instance, glucose is an 'analyte' in a biosensor designed to detect glucose.

As used herein, the term "biosensor" is a device that measures biological or chemical reactions by generating signals proportional to the concentration of an analyte in the reaction.

As used herein, the term "bioreceptor" is a molecule that specifically recognizes the analyte and is known as a bioreceptor. Enzymes, cells, aptamers, deoxyribonucleic acid (DNA) and antibodies are some examples of bioreceptors.

As used herein, the term "bio-recognition" is a process of signal generation (in the form of light, heat, pH, charge, or mass change, etc.) upon interaction of the bioreceptor with the analyte and is termed bio-recognition.

As used herein, the term "transducer" is an element that converts one form of energy into another. In a biosensor the role of the transducer is to convert the bio-recognition event into a measurable signal. This process of energy conversion is known as signalization. Most transducers produce either optical or electrical signals that are usually proportional to the amount of analyte-bioreceptor interactions.

Exploring non-invasive and periodic sensing for various biomarkers results in eccrine sweat as an ideal surrogate diagnostic biofluid. Sweat contains various physiological and metabolic biomarkers, which are indicators of human health and performance and an area of significant research interest. These biomarkers correlate with blood plasma, so candidate analytes such as glucose which requires periodic monitoring are of interest. Developing a biosensor for detecting and measuring these analytes, typically present in trace amounts in sweat requires a molecular model of biosensor with its primary constituents, viz. bioreceptor, substrate, ligand, and competing species within an appropriate eccrine sweat model. The detection of small biomolecules (< 1000 Da) such as glucose (180.156 g/mol or Da) in varying concentrations of Na and Cl ions within the physiological temperature can be aided with such a model. The necessary computational framework is required to provide insights into the intricate dynamics of biomarker detection in eccrine sweat. Presently, the medical gold standard for measuring these biomarkers via blood serum necessitates invasive procedures for sample collection and is perceived as a stress-inducing act by many subjects thereby causing erroneous results. Besides, the frequent sample collections due to the unique cycle demand frequent invasive procedures. Therefore, patient compliance is a perennial issue making sweat-based measurements particularly significant despite the comparatively low concentration of target analytes. Additionally, early detection of increased glucose levels before it culminates into diseases and appropriate control is the key to ensuring a healthy society.

**In** the present disclosure, the MD model capable of simulating various bioreceptors and target ligands in eccrine sweat environment and gold substrate is supplemented with the artificial neural network (ANN) model to identify candidate bioreceptors. This is illustrated via the candidate bioreceptor protein to further develop a wearable biosensor. The suitability of the candidate bioreceptor is validated via computing the root mean square deviation (RMSD) plots at various interaction levels and potential of mean force (PMF) of the candidate bioreceptor and the target analyte. The MD model is able to establish comparable binding energy values of various bioreceptors, study the interference with competing biomolecules (i.e. analytes) such a progesterone, testosterone, and glucose and to assess its structural stability and binding potential. The N-terminal cysteine added tothe illustrated candidate bioreceptor serves as a thiol-gold bond with the gold electrode substrates for further development as an electro-chemical biosensor. This MD model is intended to serve as a tool to aid the design of highly specific wearable biosensors for systematic development of various wearable biosensors. The model utilizes an insilico-model of an eccrine sweat to perform MD simulation. Thein-silico eccrine sweat model serves as a solvent to simulate the sweat matrix for developing wearable biosensors and reduce multiple laboratory experiments. This tool is intended to serve as a horizontal means for a broad spectrum of users for selection of bioreceptor element of wearable sensors for glucose, and other such target analytes. Further, the MD model of the present disclosure is used to simulate different sweat salt (sodium chloride/NaCl) concentrations, temperatures and their impact on the tertiary structure and corresponding binding affinity.

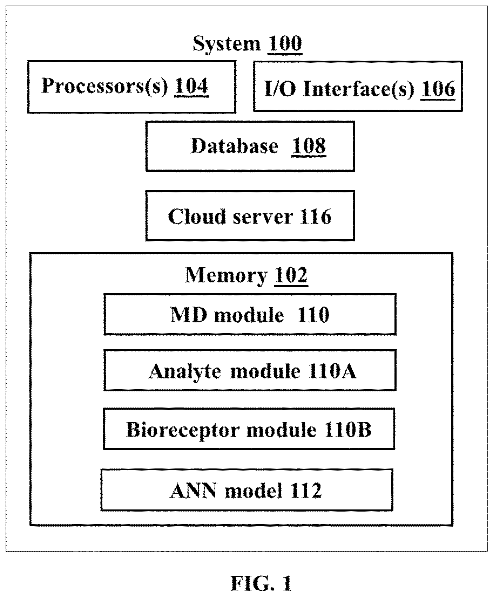

Referring now to the drawings, and more particularly to FIG. 1 through FIG. 9, where similar reference characters denote corresponding features consistently throughout the figures, there are shown preferred embodiments, and these embodiments are described in the context of the following exemplary system and/or method.

FIG. 1 illustrates an exemplary block diagram of a system 100 for identifying candidate bioreceptors using artificial neural network (ANN) based screening of a sub-set of bioreceptors obtained from molecular dynamics (MD) simulation, according to some embodiments of the present disclosure.

In an embodiment, the system 100 includes one or more processors 104, communication interface device(s) or input/output (I/O) interface(s) 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, system 100 can be implemented in a variety of computing systems, such as, laptop computers, notebooks, hand-held devices, workstations, mainframe computers, servers, a network cloud, and the like. The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) 106 can include one or more ports for connecting a number of devices such as the user terminals enabling user to communicate with system via the chat bot UI or enabling devices to connect with one another or to another server. The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, memory 102 may include a database or repository. Memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. The system 100 comprises a molecular dynamics (MD) model 110 most suitable bioreceptor for a biosensor that interacts with a given analyte of interest by means of simulating the bioreceptor and analyte interaction in an explicit solvent environment. The explicit solvent environment represents physiological state of a subject for which biosensor is to be used. The MD model 110 validates the efficacy and operability of the biosensor at various temperature and pressure conditions as well as salt concentrations of eccrine sweat. These are intended to simulate various subject pathological conditions such simulating normal body temperature and elevated temperatures caused by fever / other diseases occurs at greater than 310K (Kelvin) temperature. Also physiological state may have varied concentration of salt present in sweat dehydration conditions of the subject (10-100 mM). The MD model 110 is configurable according to the physiological state of the subject imparting an operational efficiency to the biosensor. Further, the MD model 110 reads the physical variables to be simulated. These variables include the initial positions and velocities of the atoms to be simulated, the force field parameters for said atoms, the conditions to be simulated, for example temperature and pressure, and various configuration parameters for the system, such as the number of steps to be simulated and various internal configuration parameters of the program. The MD model 110 comprises of an analyte module 110 A and a bioreceptor module 110B. The analyte module 110 A receives the analyte of interest for which the biosensor is to be designed. E.g., in an eccrine sweat environment, the analyte of interest could be glucose, cortisol or testosterone and the like. Further, the analyte module 110A executes pre-processing of the analyte that addresses suitability of the analyte for MD simulation by checking correctness in terms of scattering, baselines changes, peak shifts, noises, missing values, and several other artefacts so that the "true" chemically-relevant underlying structure can be highlighted and/or, if required, the property of interest can be predicted correctly. The bioreceptor module 110 B receives a plurality of bioreceptors, wherein the bioreceptors are selected from a bioreceptor library based on their known affinity to the analyte. The plurality of bioreceptors with known affinity towards the analyte are extracted from public repository such as protein data bank (PDB), GenBank, NCBI, PROSITE and the like or user designed bioreceptors. The bioreceptor module 110B further executes pre-processing of the plurality of bioreceptors, via the one or more hardware processors. The pre-processing involves lot many curations to offer a bioreceptor before it is allowed to be interacted with the analyte of interest. The pre-processed files are stored internally for molecular dynamics simulation. The system 100 comprises an artificial neural network (ANN) model 112. The ANN model 112 has an input layer with as many nodes as the number of input features. In an embodiment, the model processes theinput features corresponding to three RMSD plots. For example, X1, X2, ......, XN are input features. The model starts with a single hidden layer with multiple neurons. The number of neurons and the number of hidden layers are hyperparameters that can be tuned using hyper parameter tuning methods such as keras-tuner. The output layer has a single node for the classification result. If the RMSD values of all three sub-components are less than 0.2 nm, the output is 1 (desired), otherwise, it is 0 (non-desired). The memory 102 further includes a plurality of modules (not shown here) comprises programs or coded instructions that supplement applications or functions performed by the system 100 for executing different steps involved in the data rate prediction and prioritization. The plurality of modules, amongst other things, can include routines, programs, objects, components, and data structures, which perform particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by one or more hardware processors 104, or by a combination thereof. The plurality of modules can include various sub-modules (not shown).

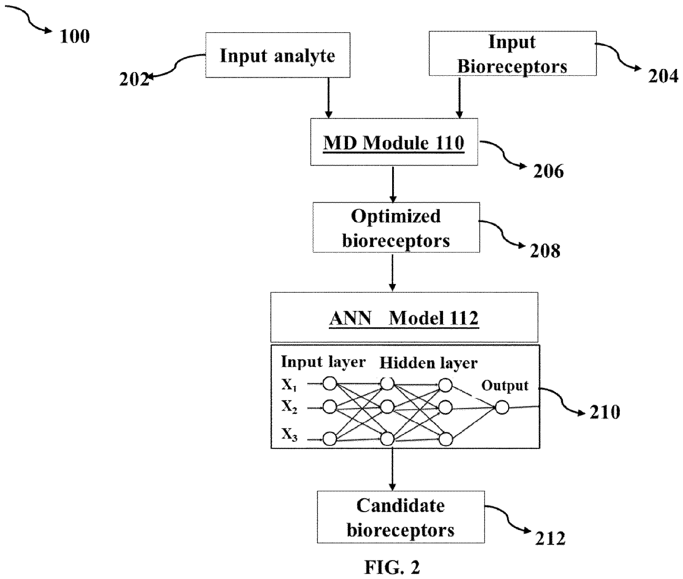

FIG. 2 is a diagram that illustrates essential components of the system 100 with sequential functionalities for identifying candidate bioreceptors using combined ensemble of molecular dynamics and artificial neural network (ANN), according to some embodiments of the present invention.

As illustrated in FIG. 2, the system 100 comprises of two major functionalities, the MD model 110 that executes simulation of analyte with the bioreceptors to screen suitable bioreceptors and, the ANN model 112 that classifies the output of MD model 110 to predict candidate bioreceptor suitable for designing as a biosensor for identification of the analyte of interest.

In an embodiment, the MD model 110 is a GROMACS package, a comprehensive molecular dynamics simulation tool for dynamics study. The MD model 110 receives the analyte of interest at 202 for which a biosensor is to be designed. Simultaneously, the MD model 110 receives plurality of bioreceptors at 204 with known affinity towards the analyte of interest. The plurality of bioreceptors is in the form of a file comprising curated bioreceptors selected for performing molecular dynamics simulation with the analyte of interest. The MD model 110 processes the analyte of interest and the bioreceptors by performing simulation in the explicit solvent environment. In the explicit-solvent method, solvent molecules are treated explicitly, i.e., interactions between all pairs of solute and solvent atoms are explicitly computed. The Particle Mesh Ewald (PME) approximation, the most commonly used explicit-solvent method for long range interactions. Periodic boundary conditions speed up these computations by imposing an artificial periodicity on the entire system and treating the system as an infinite crystal with identical repeating cells. This assumption allows for a mathematical transformation that approximates the long-range interactions very efficiently, without significant loss in accuracy. The explicit solvent model consideration mimics the physiological condition of a person at which biosensor is to be used. The model can be used to further validate efficacy and operability of the biosensor at various temperature and pressure conditions as well as salt concentrations of eccrine sweat. These are intended to simulate various subject pathological conditions such simulating normal body temperature and elevated temperatures caused by fever/ other diseases > 310K, also the concentration of salt present in sweat dehydration conditions of the subject (10-100 mM). As the system 100 receives the analyte and the bioreceptors, the MD model 110 executes simulation process at 206. At first, substrate is processed to make it suitable for the MD simulation. The substrate is modelled with Lennard jones (LJ) potential on the surface of the substrate to obtain minimized substrate surface and further subjecting the minimized substrate surface to a statistical ensemble to estimate the stability of the minimized substrate surface under dynamic conditions wherein the statistical ensemble utilized is NVT (constant number of atoms, constant volume, constant temperature). In an embodiment, gold is utilized as the substrate. During pre-processing, gold is prepared by surface design tools such as Nanocut and Avogadro. The minimized surface is taken for NVT dynamics to check its stability under dynamic conditions. Further, the candidate bioreceptor is placed on minimized gold surface and subsequently placed in a bounding box. The space left within the bounding box is filled with sweat solution (i.e. explicit solvent environment) using PACKMOL software. The bioreceptor and the pre-processed substrate forms the bioreceptor-substrate complex. The substrate holds the bioreceptor by way of providing a base to the bioreceptor by immobilizing the bioreceptor on the surface. However, the substrate and the bioreceptor does not undergo any chemical reaction except for the gold-thiol bond for immobilizing the bioreceptor. Further, the MD tool 110 performs energy minimization of the bioreceptor-substrate complex, and then NVT equilibration is performed at physiological temperature and pressure to check the stability of the bioreceptor on the surface and in 50mM NaCl solution. Next, the compatibility of the bioreceptor and the analyte is assessed. The optimized bioreceptor-analyte complex is evaluated by simulating bioreceptors with the analyte. This further screens out the bioreceptors from the initial set of bioreceptors received by the system 100 that do not show desired affinity towards the analyte. Furthermore, to check the binding ability of the bioreceptor with the analyte in in the explicit solvent environment (e.g. sweat solution), the bioreceptor along with the analyte is packed on the substrate surface and solvated with. This analyte-bioreceptor-substrate complex is further taken for energy minimization. The energy minimization is conducted under an NVT ensemble (constant Number of particles, Volume, and Temperature). This ensemble is also referred to as "isothermal-isochoric" or "canonical." The timeframe for such a procedure is dependent upon the contents of the system, but in NVT, the temperature of the system should reach a plateau at the desired value. The NVT equilibrations are carried out in two steps. At first step, position restraints are placed at the both substrate surface and the biomolecules while solvent solution isallowed to move freely so as to fill the voids. At the second step, simulation is performed without any restraints. In an embodiment, the first step is simulated for about 1 ns while the second step is simulated for about 10 ns. After this, production run is carried out for about 30 ns was and the results are analyzed. The extracted trajectories thus obtained are used to calculate properties. In an embodiment, both during the equilibration run and the production run, the temperature and pressure are regulated using suitable standards. The electrostatic and van der Waals cutoffs are set. The particle mesh Ewald (PME) summation method is used to treat long-range electrostatic interactions. The neighbour list cutoff is set and updated at a regular frequency. Therefore, minimized analyte-bioreceptor-substrate complex is obtained. To further process minimized analyte-bioreceptor-substrate complex in MD model 110, the root mean square deviation (RMSD) plots are calculated individually for the bioreceptor-substrate complex, analyte-bioreceptor complex and minimized analyte-bioreceptor-substrate complex. Further, the potential of mean force (PMF) for the minimized analyte-bioreceptor-substrate complex is calculated. The PMF is a measure of binding affinity between the analyte and the bioreceptor (substrate immobilized bioreceptor in the present disclosure). To calculate the PMF, initial configurations are generated by constraining the analyte molecule at various locations radially outwards from the centre-of-mass (CoM) from the bioreceptor using harmonic potential. Starting configurations are generated by pulling the analyte molecule from the CoM of the bioreceptor at a constant. During this process, snapshots of the system are captured when the distance between the CoM of the bioreceptor and the constrained ligand changed. The captured windows are equilibrated followed by a short NPT simulation with the analyte restrained in the radially outward direction using a harmonic force constant. The simulation trajectory is saved every 10 ps. Further, The PMF is generated using the weighted histogram analysis method (WHAM) in GROMACS. Next, the RMSD plots and the PMF plot are fed to a pre-trained ANN model 112 wherein the pre-trained ANN model 112 estimates overall structural stability and binding pocket stability of the one or more desired bioreceptor to the analyte through feature engineering to predict candidate bioreceptors at 210. The pre-trained ANN model 112 receives the X1, X2, ..., Xn input features derived from the RMSD plots of the bioreceptor-substrate, the bioreceptor-analyte and the analyte-bioreceptor-substate complexes, and the PMF values. The input layer of the ANN model 110 has as many nodes as the number of input features. RMSD plots along with their corresponding features input to the ANN model 110 by the input layer. Further the ANN model 110 has two hidden layers with three neurons each. However, hidden layers starting point can be anyone from a single hidden layer with multiple neurons. The number of neurons and the number of hidden layers are hyperparameters those can be tuned using hyper parameter tuning methods such as keras-tuner. In the output layer, the ANN model 110 have a single node, that outputs the classification result. If the RMSD values of all three sub-components are less than 0.2 nm, the output is 1 (desired), otherwise, it is 0 (non-desired). Further, appropriate activation function is assigned for each layer. In an embodiment, sigmoid activation function is utilized as the problem statement is classified as a binary classification problem. Further, ReLU activation function is used in the output layer and for input and hidden layer. The ANN model 110 is compiled by specifying the loss function, optimizer, and metrics to evaluate the model performance. The ANN model 110 is pre-trained to execute desired function. During training, the ANN model 110 is trained using the prepared dataset. During training, the model learns to map input RMSD values to the corresponding labels. After training, model performance is evaluated using separate validation dataset to ensure it generalizes well to unseen data. The trained ANN model 110 further makes prediction on new RMSD values to identify the candidate bioreceptors suitable for the analyte of interest at 212.

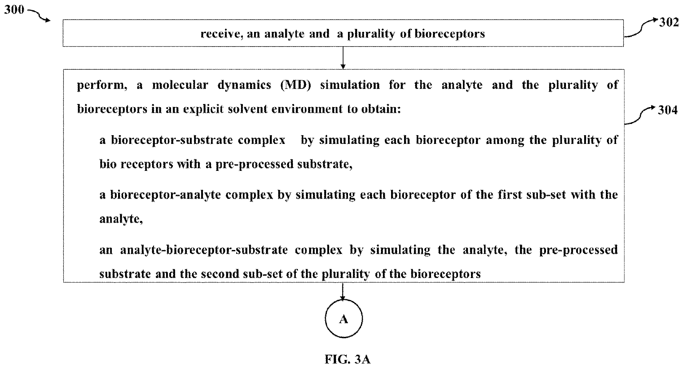

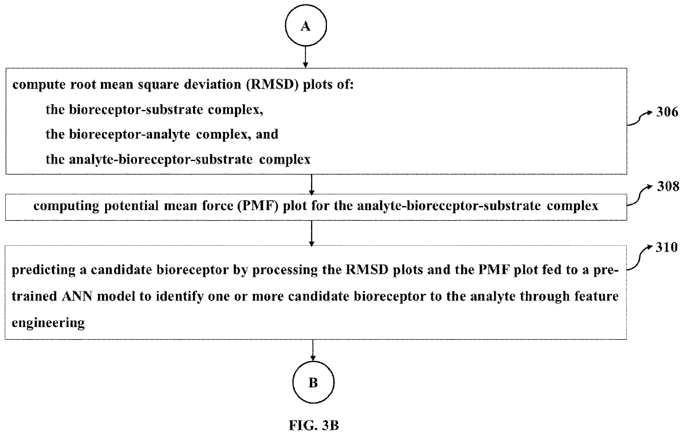

FIGS. 3A and 3B are flow diagrams of an illustrative method 300 for identifying candidate bioreceptors using MD simulation and ANN based classification, according to some embodiments of the present disclosure.

The steps of method 300 of the present disclosure will now be explained with reference to the components or blocks of the system 100 as depicted in FIG. 1 through FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously. The system 100 comprises of MD model 110 and screens the large number of bioreceptors having known affinity towards the analyte of interest. The screening is based on molecular dynamics simulation wherein interaction of the analyte with bioreceptors under the influence of explicit solvent is assessed. The screened bioreceptors are further fed tothe ANN model 112 wherein an informed decision is made on the basis of feature engineering performed by the ANN model 112 to identify candidate bioreceptors for the analyte of interest. At step 302 of the method 300, the one or more hardware processors 104 are configured to receive an analyte anda plurality of bioreceptors, wherein the bioreceptors are selected from a bioreceptor library based on their known affinity to the analyte. The bioreceptors are either natural compounds or engineered using advanced computational and biological methods. The analyte selection is based on the chemical in the biological fluid to be detected from a biosensor. In an embodiment, the analyte is glucose to be detected from an eccrine sweat sample collected from a wearable biosensor. The analyte is further processed for making it suitable for MD simulation by checking correctness in terms of scattering, baselines changes, peak shifts, noises, missing values, and several other artefacts so that the "true" chemically-relevant underlying structure can be highlighted and/or, if required, the property of interest can be predicted correctly. Next, the plurality of bioreceptors are obtained from a bioreceptor library based on their known affinity to the analyte. The plurality of bioreceptors with known affinity towards the analyte are extracted from public repository such as protein data bank (PDB), GenBank, NCBI, PROSITE and the like. The plurality of bioreceptors selected based on the binding affinity are processed by applying edits to curate the structure, orientation, and charges.

At step 304 of the method 300, the one or more hardware processors 104 are configured to perform, a molecular dynamics (MD) simulation for the analyte and the plurality of bioreceptors in an explicit solvent environment to obtain: (a) a bioreceptor-substrate complex by simulating each bioreceptor among the plurality of bioreceptors with a pre-processed substrate, (b) a bioreceptor-analyte complex by simulating each bioreceptor of the first sub-set with the analyte, and (c) an analyte-bioreceptor-substrate complex by simulating the analyte, the pre-processed substrate and the second sub-set of the plurality of the bioreceptors. The surface minimization of the substrate utilized in the immobilizing the bioreceptor is performed by minimizing the layers then subjecting the minimized layers to NVT dynamics to check its stability under dynamic conditions. The minimized substrate is then placed in a cubical bounding box and the vacant spaces of the bounding box is filled with a solvent. In an embodiment, the solvent is eccrine sweat and thus, the vacant spaces in the cubical bounding box is replaced with the eccrine sweat. This minimized substrate interacts with the one or more bioreceptors. As a result of interaction, the bioreceptors retains on the surface of the substrate by way of immobilization forming the bioreceptor-substrate complex. This is performed to ensure that the minimized substrate surface is compatible with the first sub-set of the plurality of bioreceptors received. Next, the bioreceptor-analyte complex is obtained by simulating each bioreceptor of the first sub-set with the analyte. The binding interactions between the each bioreceptor of the first sub-set and the analyte is assessed which are involved in binding affinity and specificity calculations. The stability of the bioreceptor-analyte complex is established by performing unconstrained MD simulations in the solvent environment. The bioreceptors capable of forming favorable interactions with the analyte forms the second sub-set of the plurality of bioreceptors received. The analyte and the bioreceptor, duly processed and the minimized substrate are then simulated to obtain an analyte-bioreceptor-substrate complex. Further, the NVT equilibration is carried out in two steps. In the first step, position restraints are placed at substrate surface and bioreceptor while the solvent (eccrine sweat solution) is allowed to move freely so as to fill the voids. In the second step, simulation is carried on without any restraints. After this, production run is carried out for a short period of time and the results are analyzed. The extracted trajectories are used to calculate properties. Both, during the equilibration run, and the production run, temperature and pressure are kept under control. Further, conditioning is done by adjusting electrostatic and van der Waals cutoffs and the long-range electrostatic interactions are studied. Subsequently, the cutoff values are updated at a regular interval. Therefore, a further optimized bioreceptors are obtained as a second sub-set of the plurality of bioreceptors having favorable interaction profile with the analyte.

At step 306 of the method 300, the one or more hardware processors 104 are configured to compute root mean square deviation (RMSD) plots of: (a) the bioreceptor-substrate complex, (b) the bioreceptor-analyte complex, and (c) the analyte-bioreceptor-substrate complex. The RMSD plots serves as a key metric to quantify the deviation of a molecular structure from its initial configuration. The RMSD plots are used for measuring the difference between the backbones of a protein from its initial structural conformation to its final position. The stability of the protein relative to its conformation can be determined by the deviations produced during the course of its simulation. The smaller the deviations, the more stable the protein structure. The correlation between the minimal changes in bioreceptor conformation, and the consistently low RMSD values presented reinforces the stability of the bioreceptor on the gold surface in the simulated solvent environment.

At step 308 of the method 300, the one or more hardware processors 104 are configured to compute potential of mean force (PMF) plot for the analyte-bioreceptor-substrate complex. The PMF in the MD simulations assesses the energy changes as a function of specific reaction coordinate parameter like the energy changes as a function of the distance between two residues, or as a force required to pull the protein molecule apart. By computing local energy barriers along a binding/unbinding path, the PMF provides thermodynamic details for molecular recognition.

At step 310 of the method 300, the one or more hardware processors 104 are configured to predict a candidate bioreceptor by processing the RMSD plots and the PMF plot fed to a pre-trained ANN model to identify one or more candidate bioreceptor to the analyte through feature engineering. Next, the RMSD plots and the PMF plot are fed to a pre-trained ANN model 112 wherein the pre-trained ANN model 112 estimates overall structural stability and binding pocket stability of the one or more desired bioreceptor to the analyte through feature engineering to predict candidate bioreceptors from the second sub-set of the plurality of bioreceptors. The pre-trained ANN model 112 receives the X1, X2, ..., Xn input features derived from the RMSD plots of the bioreceptor-substrate, the bioreceptor-analyte and the analyte-bioreceptor-substate complexes, and the PMF values. The input layer of the ANN model 110 has corresponding input features of the RMSD plots. Further the ANN model 110 has two hidden layers with three neurons each. The hidden layers having multiple neurons are hyperparameters tuned using hyper parameter tuning methods. In the output layer, the ANN model 110 have a single node, that outputs the classification result. If the RMSD values of all three sub-components are less than 0.2 nm, the output is 1 (desired), otherwise, it is 0 (non-desired). Further, appropriate activation function is assigned for each layer. In an embodiment, sigmoid activation function is utilized as the problem statement is classified as a binary classification problem. Further, ReLU activation function is used in the output layer and for input and hidden layer. The ANN model 110 is compiled by specifying the loss function, optimizer, and metrics to evaluate the model performance. The ANN model 110 is pre-trained to execute desired function. During training, the ANN model 110 is trained using the prepared dataset. During training, the model learns to map input RMSD values to the corresponding labels. After training, model performance is evaluated using separate validation dataset to ensure it generalizes well to unseen data. The trained ANN model 110 further makes prediction on new RMSD values to identify the candidate bioreceptors suitable for the analyte of interest.

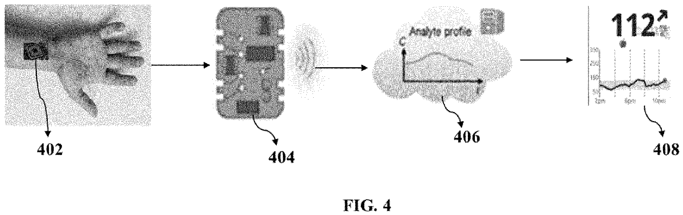

FIG. 4 illustrate a candidate bioreceptor-based device sensing a biomarker in a test sample, according to some embodiments of the present disclosure.

As illustrated in FIG. 4, the biosensor comprising the candidatebioreceptor402 is in contact with the skin so as to receive the test sample. The biosensor may be in the form of a wearable device which may be worn onto body parts, such as wrist band to be worn on the wrist and the like. The test sample contacts the transduction unit 404 to perceive the test sample for analysis purpose. Transduction units may be one of electrochemical, optical, thermal, or gravimetric sensing techniques. In a preferred embodiment of the present disclosure, the electrochemical based transduction unit is configured to process the test sample. The sample once processed by the transduction unit 404, signals thus generated are processed at data processing unit 406. The data processing unit may comprise of one or more amplifiers, processors, signal converters and data communication modules. Based on data processing the outcome of analysis as the concentration of the analyte in the test sample is displayed by a display device 408.

According to an embodiment of the present invention, the candidate bioreceptor is in the form of a biosensor comprising: the candidate bioreceptor; and a transducer, wherein the candidate bioreceptor binds with the analyte, and a detectable signal is transduced through the transducer. The biosensor comprises the candidate bioreceptor screened using the method disclosed in the present disclosure and offers an electrochemical biosensor that comprises of a substrate. A wide variety of materials may be used as substrates for supporting electrodes including paper, polyethylene teraphalate (PET), kapton film, cyclein olefin copolymer and cellulose, making them favorable for flexible electronics. The substrate layer contacting the candidate bioreceptor is made of gold, graphene etc and is obtained by pre-processing the substrate under MD simulation and NVT dynamics to obtain energy minimized gold substrate. The analyte of interest (i.e. β-D-glucose in this case) binds to the candidate bioreceptor to enable electrochemical sensing of the analyte of interest. According to an embodiment of the present disclosure, the biosensor further comprises of a transducer, wherein the candidate bioreceptor transduces detectable signal to a substrate attached to the transducer, and wherein a signal transduced by the candidate bioreceptor bound to the analyte differs from a signal transduced by the candidate bioreceptor when the candidate bioreceptor is not bound to the analyte.

According to an embodiment of the present disclosure, the biosensor comprising the candidate bioreceptor is in contact with the skin so as to receive the test sample. The biosensor may be in the form of a wearable device which may be worn onto body parts, such as wrist band to be worn on the wrist and the like. The test sample contacts a transduction unit of the biosensor to perceive the test sample for analysis purpose. Transduction units may be one of electrochemical, optical, thermal or gravimetric sensing techniques. In a preferred embodiment of the present disclosure, the electrochemical based transduction unit is configured to process the test sample. The sample once processed by the transduction unit, and the signals thus generated are processed by a data processing unit. The data processing unit may comprise of one or more amplifiers, processors, signal converters and data communication modules. Based on data processing the outcome of analysis as the concentration of the analyte in the test sample is displayed by a display device.

According to an embodiment of the present disclosure, a β-D-glucose, the analyte of interest is a biomarker for dysglycemic disorders and is identified in the plurality of biofluids such as sweat, urine, saliva, blood and the like. According to an embodiment of the present disclosure, a β-D-glucose is identified in the eccrine sweat as a non-invasive means of identification and monitoring the dysglycemic disorders. The candidate bioreceptor 1HKC 172:294 utilized in analyte identification is identified using combined MD simulation and ANN based screening according to the method of the present disclosure.

According to an embodiment of the present invention, the candidate bioreceptor detects the analyte in a test sample by a process comprising steps: (a) the test sample comprising a biofluid, wherein the biofluid potentially contains the analyte; (b) a biosensor comprising the candidate bioreceptor; (c) contacting the test sample with a surface of the biosensor; (d) permitting signal generation to occur as the analyte contacts the candidate bioreceptor of the biosensor; and (e) detecting the presence or amount of the analyte in the test sample using a detection assembly.

According to an embodiment, the biosensor is in the form of a wearable device wherein the wearable device comprising the candidate bioreceptor (i.e. 1HKC 172:294) identified by combined MD simulation and ANN based screening capable of sensing glucose is illustrated. The wearable device comprises of a biosensor array, a power source, a data processor and a data transmitter. The biosensor array comprising the candidate bioreceptor along a circumference of the wearable device including at least one electromagnetic energy emitter and at least one electromagnetic energy receiver. A power source is provided to the wearable device. A data processing unit receives data from the electromagnetic energy receiver which is analyzed in order to measure glucose level of a subject; and a data transmitter that transmits the signal that are ultimately displayed on a display of the wearable device. The device is highly integrated and does not need external auxiliary equipment. The glucose concentration can be obtained through an LCD screen mounted on a wearable device. Through experimental detection, the detection device designed has high detection accuracy. In particular, it can obtain the glucose content in the human body through noninvasive detection, which shows the broad prospect of the application of the detection device in the field of health examination and monitoring.

### USE CASE: IDENTIFYING CANDIDATE BIORECEPTOR FOR GLUCOSE ANALYTE USING COMBINED MD SIMULAITON AND ANN BASED SCREENING:

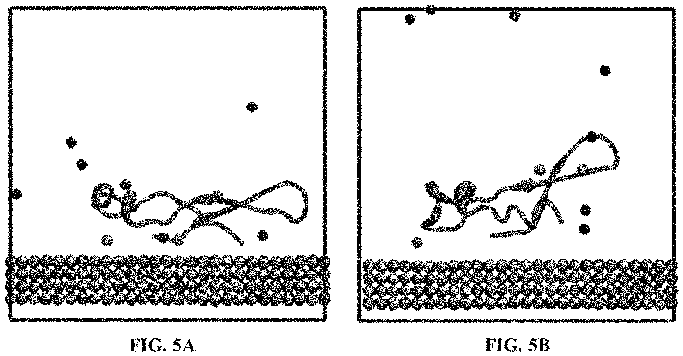

An example scenario depicting the method of identifying candidate bioreceptors suitable for designing glucose biosensor by the disclosed system 100 is described with reference to the FIGS. 5-9. Bioreceptor identification for glucose analyte is performed so that a biosensor can be designed that can detect glucose in the eccrine sweat. Glucose is taken as an analyte and is pre-processed to ensure completeness withing the structure. Generally, the ligand lacks hydrogen atoms, so hydrogen atoms are added to the ligand and rotatable bonds are defined that are used for MD simulation. A plurality of bioreceptors that are expressed in dysglycemic disorders are selected from a public repository (PDB). The naturally available proteins like glucose/ galactose binding protein (GGBP), sugar binding proteins, transport proteins, lectins, etc were imported. Further, selected proteins are taken for pre-processing. The pre-processing involves lot many curations to offer a protein before it can be docked. All the non-protein parts are identified and removed from the protein, such as ligands (HETATM), ions (CL, NA etc) and water (SOL). Then, the missing hydrogens are added. Further, appropriate force fields were selected, Kollman charges were added. The pre-processed files are stored internally as PDBQT files compatible with the GROMACS, a MD software. At first, gold bulk is taken for energy minimization a 5.3 nm x 5.3 nm surface having 4-atomic layers is created using Nanocut and Avogadro. The surface is minimized and then subjected to NVT dynamics to check its stability under dynamic conditions. Further, the bioreceptors are placed on minimized gold substrate surface and subsequently placed in a cubic box of size 5.3 nm and filled the vacant spaces of cubic box with 50 mM NaCl (sweat) solution using PACKMOL software. This system is first energy minimized and then NVT equilibration is done at 310 K (physiological) temperature and atmospheric pressure 1 bar to check the stability of the bioreceptor on the surface and in 50mM NaCl solution. This forms the first sub-set of the plurality of bioreceptors selected based on favorable interaction with the minimized substrate.

The first sub-set of the bioreceptors from the plurality of bioreceptors is thus formed based on NVT dynamics. Furthermore, to check the binding ability of bioreceptors of the first sub-set with the analyte in sweat solution, energy minimization and NVT dynamics are performed to identify the bioreceptors having favorable interaction with the analyte.. This forms the second sub-set of the plurality of bioreceptors selected based on favorable interaction with the analyte. The characteristics of the bioreceptors of the second sub-set is presented in Table-1. Each bioreceptor of the second sub-set along with target analyte is packed on gold surface (the substrate) and solvated with sweat solution. The system is then energy minimized. All the NVT equilibration is carried out in two steps. **In** the first step, position restraints are placed at gold surface and biomolecules while sweat solution is allowed to move freely so as to fill the voids and in the second step, simulation is performed without any restraints. The step one is simulated for 1 ns while step two is simulated for 10 ns. After step two, production run for 30 ns is carried out and the results are analyzed. The extracted trajectories are used to calculate properties. During the equilibration run, the temperature and pressure is controlled with the Berendsen thermostat and barostat with time constants of 1ps and 5ps, respectively. **In** the production run, the temperature and pressure are controlled by the Nosé-Hoover thermostat and the Parrinello-Rahman barostat, applying time constants of 2ps and 5ps, respectively. The electrostatic and van der Waals cutoffs are set at 1.2 nm. The particle mesh Ewald (PME) summation method is used to treat long-range electrostatic interactions. The neighbour list cutoff is set at 1.2 nm and updated every tenth step.

**Table-1**

| Top 5 bioreceptors screened as the second sub-set of the plurality of bioreceptors: | | | | | |
|---|---|---|---|---|---|
| **Protein ID** | **Bioreceptor Interactions** | **Atom-atom interactions and corresponding bond length (Å)** | **Average RMSD Values (Å)** | **ANN feature based on which decision is made (Feature engineering)** | **ANN Classifier Select (1) Reject (0)** |
| 1HKC | 1HKC (172:294) - Gold Substrate | | 1.5 | | 1 |
| | 1HKC (172:294) - Glucose | 1. THR172-O1 2.36 | 1.2 | | |
| | | 2. LYS173-O6 2.17 | | | |
| | | 3. ASN208-O3 2.04 | | Number of interactions, Individual bond lengths, | |
| | | 4. ASN208-O4 2.39 | | | |
| | | 5. ASP209-O4 1.92 | | | |
| | | 6. ASP209-O6 1.73 | | | |
| | | 7. ASN235-O41.98 | | | |
| | | 8. GLU260-O22.01 | | | |
| | | 9. GLU260-O32.00 | | RMSD values | |
| | | 10. GLU294-O11.96 | | | |
| | 1HKC (172:294) - Glucose - Gold Substrate | | 1.0 | | |
| 6BGC | 6BGC (19:172) - Gold Substrate | | 1.8 | Number of interactions, Individual bond lengths, | 1 |
| | 6BGC (19:172) - Glucose | 1. Asn19-O5 1.99 | 1.7 | | |
| | | 2. Asn19-O6 1.94 | | | |
| | | 3. Arg20-O6 2.19 | | | |
| | | 4. Arg95-O2 1.84 | | RMSD values | |
| | | 5. Asp195-O2 1.77 | | | |
| | | 6. Asp195-O3 1.86 | | | |
| | | 7. Asn172-O3 2.28 | | | |

| | | | | | |
|---|---|---|---|---|---|
| | 6BGC (19:172) - Glucose - Gold Substrate | | 1.6 | | |
| 1EXI | 1EXI (95:207) - Gold Substrate | | 2.5 | | 0 |
| | 1HKC (172:294) - Glucose | 1. Asp95-O32.19 | 2.1 | Number of interactions, Individual bond lengths, | |
| | | 2. Asp95-O42.08 | | | |
| | | 3. Asp95-O51.92 | | | |
| | | 4. His207-O22.25 | | | |
| | 1HKC (172:294) - Glucose - Gold Substrate | | 2.1 | RMSD values | |
| 1HIZ | 1HIZ (237:358) - Gold Substrate | | 1.4 | | 1 |
| | 1HIZ (237:358) - Glucose | 1. His237-O4 2.35 | 1.1 | | |
| | | 2. Gln239-O41.79 | | Number of interactions, Individual bond lengths, | |
| | | 3. Asp268-O61.88 | | | |
| | | 4. Gly273-O21.96 | | | |
| | | 5. Gly273-O31.93 | | | |
| | | 6. Arg329-O12.63 | | | |
| | | 7. Arg329-O52.58 | | | |
| | | 8. Asp358-O12.36 | | RMSD values | |
| | 1HIZ (237:358) - Glucose - Gold Substrate | | 1.0 | | |
| 1GWW | 1GWW (40:146) - Gold Substrate | | 1.6 | Number of interactions, Individual bond lengths, | 1 |
| | 1GWW (40:146) - Glucose | 1. Ile40-O62.10 | 1.4 | | |
| | | 2. His142-O12.46 | | RMSD values | |
| | | 3. Glu145-O13.06 | | | |
| | | 4. Glu145-O3 2.02 | | | |
| | | 5. Glu145-O4 2.12 | | | |
| | | 6. Glu145-O5 2.02 | | | |
| | | 7. Glu145-O6 2.61 | | | |
| | | 8. Glu146-O11.92 | | | |
| | | 9. Glu146-OH1 2.81 | | | |
| | | 10. Glu146-O22.06 | | | |
| | 1GWW (40:146) - Glucose - Gold Substrate | | 1.2 | | |

The top five bioreceptors forming the second-set are further assessed by computing the RMSD plots for bioreceptor-substrate, analyte-bioreceptor and analyte-bioreceptor-substrate complexes for each bioreceptor. Further, potential of mean force (PMF) for the target analyte and selected bioreceptor is calculated. The PMF is a measure binding affinity between analyte and bioreceptor. To calculate the PMF, initial configurations are generated by constraining the analyte molecule at various locations radially outwards from centre-of-mass (CoM) from bioreceptor using harmonic potential. Starting configurations were generated by pulling the analyte molecule from the CoM of the bioreceptor until 4.5 nm at a constant velocity of 0.048 nm/ps. During this process, snapshots of the system are captured when the distance between the CoM of the bioreceptor and the constrained ligand changed by 0.2 nm. The 25 captured windows are equilibrated for 1 ns, followed by 3 ns of NPT simulation with the analyte restrained in the radially outward direction using a harmonic force constant of 1000 kJ mol-1 nm-2 . The simulation trajectory is saved every 10 ps. The PMF is generated using the weighted histogram analysis method (WHAM), as implemented using the gmx wham command in GROMACS. The ANN model 110 receives the RMSD plots for the bioreceptor-substrate, the bioreceptor-analyte and the analyte-bioreceptor-substrate complexes for the binary classifier and provides an output for stability of the candidate bioreceptor on the basis of RMSD plot feature such as RMSD values (< 2 Å) and their mean, variance and standard deviation and assign "select or 1" status as per pre-trained values. Otherwise, the candidate bioreceptor is assigned "reject or 0" status. Subsequently, the ANN model 110 (which is multi-class classification model) considers the PMF plots as the input with features such as ΔG (binding energy) value, area under the PMF curve and slope of the PMF curve with preassigned weights to provides ranking of the various candidate bioreceptors.

The RMSD plots of the bioreceptor-substrate, the bioreceptor-analyte and the analyte-bioreceptor-substate complexes, and the PMF values of the bioreceptors of the second sub-set are fed to the pre-trained ANN model. The input layer of the ANN model having input features corresponding to the RMSD plots, the two hidden layers with three neurons each and the output layer with a single node outputs the classification result. The analyte-bioreceptor-substrate complex with RMSD values of all three sub-components >0.2 nm are suggested as 'select' (Output as 1), otherwise, it is 'reject' (Output as 0). The bioreceptor suitable for analyte-bioreceptor-substrate complex based on ANN is presented below in Table-2.

**Table-2**

| **Top 3 bioreceptors screened based on combined output of MD and ANN:** | | | | | | |
|---|---|---|---|---|---|---|
| **Protein ID** | **Interactions** | **Simulation time (ns)** | **RMSD Values (Å)** | **PMF (A-B-S)** (kcal/ mol) | **ANN feature based on which decision is made (Feature engineering)** | **ANN Model Selection score (1-10) where 10 is the best candidate** |
| 1HKC | 1HKC (172:294) - Gold Substrate | 10 | 1.5 | -7.38 | PMF value, Area under the curve, Slope, ΔG (binding energy) | 10 |
| | 1HKC (172:294) - Glucose | 10 | 1.2 | | Cumulative RMSD values, standard deviation, variance, mean | |
| | 1HKC (172:294) - Glucose - Gold Substrate | 30 | 1.0 | | | |
| 6BGC | 6BGC (19:172) - Gold Substrate | 10 | 1.8 | -6.19 | PMF value, Area under the curve, Slope, ΔG (binding energy) | 9 |
| | 6BGC (19:172) - Glucose | 10 | 1.7 | | Cumulative RMSD values, standard deviation, variance | |
| | 6BGC (19:172) - Glucose - Gold Substrate | 30 | 1.6 | | | |
| 1HIZ | 1HIZ (237:358) - Gold Substrate | 10 | 1.4 | -5.92 | PMF value, Area under the curve, Slope, ΔG (binding energy) | 8 |
| | 1HIZ (237:358) - Glucose | 10 | 1.1 | | Cumulative RMSD values, standard deviation, variance | |
| | 1HIZ (237:358) - Glucose - Gold Substrate | 30 | 1.0 | | | |

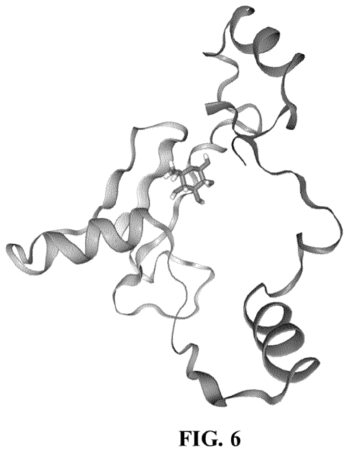

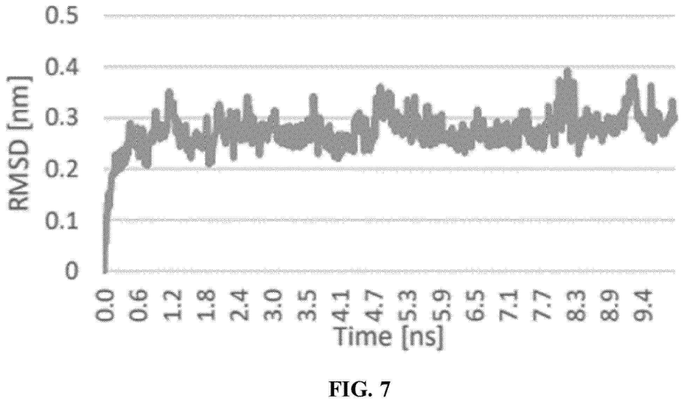

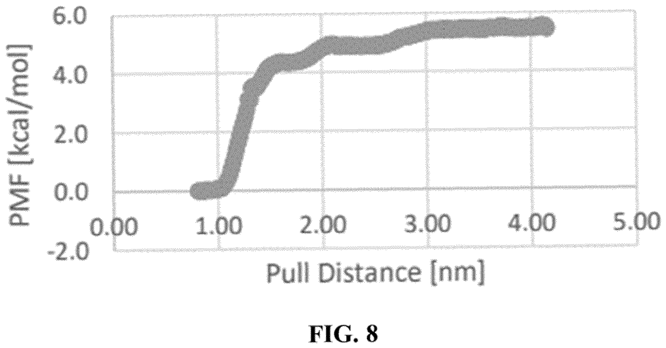

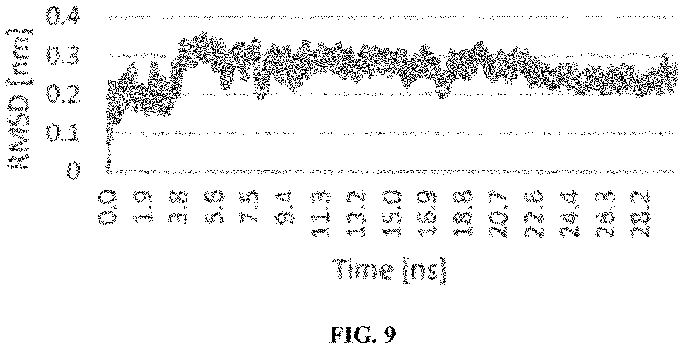

Therefore, based on input RMSD values, the ANN model predicted 1HKC (172:294) as a suitable bioreceptor for the glucose analyte. The initial and the final conformations of 1HKC (172:294) bioreceptor on the surface of the gold substrate is presented in FIGS. 5A-5B. A bond is created between one of the gold atoms on the surface with thiol group of cysteine. FIG. 6 represents the candidate bioreceptor (1HKC 172 : 294) complexed with glucose analyte in the center. The MD simulation results presented in TABLE -1 shows the resilience of the bioreceptor when exposed to the gold surface in the presence of a simulated sweat environment. The bioreceptor demonstrates a consistent conformational profile, suggesting that the bioreceptor undergoes minimal structural changes over the simulation. This stability is crucial for its applications in biosensing, as it implies that the bioreceptor can maintain its structural integrity in realistic conditions. This result is also significant as it suggests that the gold surface provides a stable substrate, and the simulated sweat environment does not induce substantial perturbations in the bioreceptor structure. Additionally, RMSD of 1HKC (172:294)bioreceptor on the surface is displayed in FIG. 7. The RMSD serves as a key metric to quantify the deviation of a molecular structure from its initial configuration. It is evident from FIG. 7 that RMSD < 0.2 nm implying that bioreceptor maintains a stable and well-defined structure on the surface. The correlation between the minimal changes in bioreceptor conformation, as observed in FIG. 5, and the consistently low RMSD values presented in FIG. 7 reinforces the stability of the bioreceptor on the gold surface in the simulated sweat environment. Subsequently, the potential of mean force (PMF) or binding affinity of ligand with bioreceptor was calculated using the umbrella sampling technique. The PMF profile for the ligand and bioreceptor is shown in FIG. 8, where z = 0 corresponds to the centre of the bioreceptor, and z = 4.5 nm radially away from the bioreceptor. The binding affinity was calculated to be approximately 6 kcal/mol with average RMSD of < 0.2 nm FIG. 9 over the entire simulation of 30 ns. It is observed that the tertiary structure of the selected bioreceptor does not significantly alter in the proximity of gold substrate layers and ensures that the ligand remains bound to the bioreceptor over the entire duration of the simulation. This is presented in the 10 ns simulation where the RMSD is within 0.2 nm as presented in FIG. 9 and of particular significance during development of electro-chemical bioreceptor biosensors.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein addresses unresolved problem of identifying candidate bioreceptor utilizing combined MD simulation and ANN based screening. The disclosed method is a systematic approach of screening a large number of bioreceptors having known affinity towards the analyte of interest and identifying suitable candidate bioreceptors that forms energetically favorable, and physico-chemically feasible interactions with the analyte. The disclosed system simulates various candidate bioreceptors and target ligands in the eccrine sweat environment. The suitability of the candidate bioreceptor is validated via computing RMSD plots of various interactions occurring between analyte, bioreceptor and the substrate, and the PMF of the bioreceptor complexed with analyte and the substrate. The RMSD plots and the PMF values are input to the ANN model that further predicts the candidate bioreceptor through feature engineering. The present disclosure involves utilizing the candidate bioreceptor in designing the biosensor capable of detecting analyte of interest in the biofluid.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300) for identifying candidate bioreceptors, the method comprising:
receiving (302), via one or more hardware processors, an analyte, and a plurality of bioreceptors, wherein the plurality of bioreceptors are selected from a bioreceptor library based on their known affinity to the analyte;
performing (304), via the one or more hardware processors, a molecular dynamics (MD) simulation for the analyte and the plurality of bioreceptors in an explicit solvent environment to obtain:
a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors,
a bioreceptor-analyte complex by simulating each bioreceptor of the first sub-set with the analyte to obtain a second sub-set of the plurality of the bioreceptors, and
an analyte-bioreceptor-substrate complex by simulating the analyte, the pre-processed substrate and the second sub-set of the plurality of the bioreceptors to obtain one or more stable analyte-bioreceptor-substate complex;
computing (306), via the one or more hardware processors, root mean square deviation (RMSD) plots of: the bioreceptor-substrate complex, the bioreceptor-analyte complex, and the analyte-bioreceptor-substrate complex;
computing (308), via the one or more hardware processors, potential of mean force (PMF) plot for the analyte-bioreceptor-substrate complex; and
predicting (310), via the one or more hardware processors, a candidate bioreceptor by processing the RMSD plots and the PMF plot features fed to a pre-trained ANN model, wherein the pre-trained ANN model estimates overall structural stability and binding pocket affinity of the one or more desired bioreceptor to the analyte through feature engineering.

2. The method as claimed in claim 1, wherein the pre-processed substate is modelled with Lennard jones (LJ) potential on the surface of the substrate to obtain minimized substrate surface, and further subjecting the minimized substrate surface to a statistical ensemble to estimate the stability of the minimized substrate surface under dynamic conditions wherein the statistical ensemble utilized is number of atoms constant volume, constant temperature (NVT).

3. The method as claimed in claim 1, wherein the explicit solvent environment is configurable according to the physiological state of the subject imparting an operational efficiency to the biosensor.

4. The method as claimed in claim 1, wherein the candidate bioreceptor is in the form of a biosensor comprising the candidate bioreceptor, and a transducer, wherein the candidate bioreceptor binds with the analyte, and a detectable signal is transduced through the transducer.

5. The method as claimed in claim 1, wherein a biosensor comprising the candidate bioreceptor detects the analyte in a test sample by a process comprising steps:
contacting the test sample with a surface of the biosensor wherein the test sample comprising a biofluid and, wherein the biofluid potentially contains the analyte;
permitting signal generation to occur as the analyte contacts the candidate bioreceptor of the biosensor; and
detecting the presence or amount of the analyte in the test sample using a detection assembly.

6. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive, an analyte, and
a plurality of bioreceptors, wherein the plurality of bioreceptors are selected from a bioreceptor library based on their known affinity to the analyte;
perform, a molecular dynamics (MD) simulation for the analyte and the plurality of bioreceptors in an explicit solvent environment to obtain:
a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors,
a bioreceptor-analyte complex by simulating each bioreceptor of the first sub-set with the analyte to obtain a second sub-set of the plurality of the bioreceptors, and
an analyte-bioreceptor-substrate complex by simulating the analyte, the pre-processed substrate and the second sub-set of the plurality of the bioreceptors to obtain one or more stable analyte-bioreceptor-substate complex;
compute, root mean square deviation (RMSD) plots of:
the bioreceptor-substrate complex,
the bioreceptor-analyte complex, and
the analyte-bioreceptor-substrate complex;
compute, potential of mean force (PMF) plot for the analyte-bioreceptor-substrate complex; and
predict, a candidate bioreceptor by processing the RMSD plots and the PMF plot features fed to a pre-trained ANN model wherein the pre-trained ANN model estimates overall structural stability and binding pocket affinity of the one or more desired bioreceptor to the analyte through feature engineering.

7. The system as claimed in claim 6, wherein the pre-processed substate is modelled with Lennard jones (LJ) potential on the surface of the substrate to obtain minimized substrate surface and further subjecting the minimized substrate surface to a statistical ensemble to estimate the stability of the minimized substrate surface under dynamic conditions wherein the statistical ensemble utilized is number of atoms constant volume, constant temperature (NVT).

8. The system as claimed in claim 6, wherein the explicit solvent environment is configurable according to the physiological state of the subject imparting an operational efficiency to the biosensor.

9. The system as claimed in claim 6, wherein the candidate bioreceptor is in the form of a biosensor comprising the candidate bioreceptor, and a transducer, wherein the candidate bioreceptor binds with the analyte, and a detectable signal is transduced through the transducer.

10. The system as claimed in claim 6, wherein a biosensor comprising the candidate bioreceptor detects the analyte in a test sample by a process comprising steps:
contacting the test sample with a surface of the biosensor wherein the test sample comprising a biofluid and, wherein the biofluid potentially contains the analyte;
permitting signal generation to occur as the analyte contacts the candidate bioreceptor of the biosensor; and
detecting the presence or amount of the analyte in the test sample using a detection assembly.

11. One or more non-transitory machine-readable information storage mediums comprising one or more instructions which when executed by one or more hardware processors cause:
receiving an analyte, and
a plurality of bioreceptors, wherein the plurality of bioreceptors are selected from a bioreceptor library based on their known affinity to the analyte;
performing a molecular dynamics (MD) simulation for the analyte and the plurality of bioreceptors in an explicit solvent environment to obtain:
a bioreceptor-substrate complex by simulating each bioreceptor among the plurality of bioreceptors with a pre-processed substrate, wherein one or more bioreceptors, among the plurality of bioreceptors, capable of forming a complex with the pre-processed substrate are identified as a first sub-set of the plurality of the bioreceptors,
a bioreceptor-analyte complex by simulating each bioreceptor of the first sub-set with the analyte to obtain a second sub-set of the plurality of the bioreceptors, and
an analyte-bioreceptor-substrate complex by simulating the analyte, the pre-processed substrate and the second sub-set of the plurality of the bioreceptors to obtain one or more stable analyte-bioreceptor-substate complex;
computing root mean square deviation (RMSD) plots of the bioreceptor-substrate complex, the bioreceptor-analyte complex, and the analyte-bioreceptor-substrate complex;
computing potential of mean force (PMF) plot for the analyte-bioreceptor-substrate complex; and
predicting a candidate bioreceptor by processing the RMSD plots and the PMF plot features fed to a pre-trained ANN model, wherein the pre-trained ANN model estimates overall structural stability and binding pocket affinity of the one or more desired bioreceptor to the analyte through feature engineering.

12. The one or more non-transitory machine-readable information storage mediums as claimed in claim 11, wherein the pre-processed substate is modelled with Lennard jones (LJ) potential on the surface of the substrate to obtain minimized substrate surface, and further subjecting the minimized substrate surface to a statistical ensemble to estimate the stability of the minimized substrate surface under dynamic conditions wherein the statistical ensemble utilized is number of atoms constant volume, constant temperature (NVT).

13. The one or more non-transitory machine-readable information storage mediums as claimed in claim 11, wherein the explicit solvent environment is configurable according to the physiological state of the subject imparting an operational efficiency to the biosensor.

14. The one or more non-transitory machine-readable information storage mediums as claimed in claim 11, wherein the candidate bioreceptor is in the form of a biosensor comprising the candidate bioreceptor, and a transducer, wherein the candidate bioreceptor binds with the analyte, and a detectable signal is transduced through the transducer.

15. The one or more non-transitory machine-readable information storage mediums as claimed in claim 11, wherein a biosensor comprising the candidate bioreceptor detects the analyte in a test sample by a process comprising steps:
contacting the test sample with a surface of the biosensor wherein the test sample comprising a biofluid and, wherein the biofluid potentially contains the analyte,
permitting signal generation to occur as the analyte contacts the candidate bioreceptor of the biosensor, and
detecting the presence or amount of the analyte in the test sample using a detection assembly.
